# FASCICULE DE BREVET EUROPEEN

(11) **EP 2 493 573 B1**
(45) Date de publication et mention de la délivrance du brevet: **25.12.2013**
(21) Numéro de dépôt: 10788113.8
(22) Date de dépôt: 29.10.2010
(51) Int. Cl.: A61K 31/4706, A61K 31/555, A61K 33/26, A61P 33/06

(54) **UTILISATION DE LA FERROQUINE DANS LE TRAITEMENT DU PALUDISME**
VERWENDUNG VON FERROQUIN ZUR BEHANDLUNG VON MALARIA
USE OF FERROQUINE IN THE TREATMENT OF MALARIA

(30) Priorité: 30.10.2009 FR 0905212
(43) Date de publication de la demande: 05.09.2012
(73) Titulaire: SANOFI, 75008 Paris (FR)
(72) Inventeur: FRAISSE, Laurent, F-75013 Paris (FR); STRUXIANO, Annie, F-75013 Paris (FR)
(74) Mandataire: Werner, Alain Henri
(86) Numéro de dépôt international: PCT/FR2010/052331
(87) Numéro de publication internationale: WO 2011/051634

(56) Documents cités:
- "Annex 10. Treatment of Plasmodium Vivax, P. ovale and P. Malariae infections", 1 janvier 2006 (2006-01-01), GUIDELINES FOR THE TREATMENT OF MALAIRA, WORLD HEALTH ORGANIZATION, PAGE(S) 225 - 239, XP009134036, ISBN: 9789241546942 le document en entier
- BARENDS MARION ET AL: "In vitro activity of ferroquine (SSR 97193) against Plasmodium falciparum isolates from the Thai-Burmese border", MALARIA JOURNAL, BIOMED CENTRAL, LONDON, GB LNKD- DOI:10.1186/1475-2875-6-81, vol. 6, no. 1, 27 juin 2007 (2007-06-27), page 81, XP021029789, ISSN: 1475-2875
- FOUDA MOHAMMAD F R ET AL: "On the medicinal chemistry of ferrocene", APPLIED ORGANOMETALLIC CHEMISTRY, vol. 21, no. 8, août 2007 (2007-08), pages 613-625, XP002583860, ISSN: 0268-2605
- OLLIARO P ET AL: "The global portfolio of new antimalarial medicines under development", CLINICAL PHARMACOLOGY AND THERAPEUTICS, NATURE PUBLISHING GROUP, US, vol. 85, no. 6, 1 juin 2009 (2009-06-01), pages 584-595, XP009133933, ISSN: 0009-9236 [extrait le 2009-04-29]
- Anonymous: "Plasmodium", Wikipedia, the free encyclopedia, 25 mai 2010 (2010-05-25), pages 1-22, XP002583858, Extrait de l'Internet: URL:http://en.wikipedia.org/wiki/Plasmodiu m [extrait le 2010-05-26]

## Description

La présente invention se rapporte à la mise en oeuvre d'actifs utiles pour la prévention et/ou le traitement d'infections par le parasite *Plasmodium vivax* et, plus généralement, par un parasite du genre *Plasmodium,* dont le cycle de vie comprend une phase de latence hépatique chez l'hôte humain.

Plus précisément, la présente invention concerne la mise en oeuvre de la ferroquine ou son métabolite N-déméthylé à cet effet.

Le paludisme est l'une des premières causes infectieuses de mortalité au monde et touche annuellement plus de 5 000 000 de personnes, parmi lesquelles 3 000 000 décèdent chaque année.

Ce fléau touche principalement l'Afrique sub-saharienne, l'Asie du Sud-Est et l'Amérique Latine.

On distingue généralement quatre espèces principales de Plasmodium, responsables de la transmission du *paludisme* : *Plasmodium falciparum*, *Plasmodium vivax*, *Plasmodium ovale* et *Plasmodium malariae*, les deux premières étant les plus répandues.

Les parasites *P. falciparum* et *P. vivax* se distinguent l'un de l'autre en terme de couverture géographique et au niveau de leur cycle de développement dans l'hôte humain.

*P. vivax* constitue l'espèce plasmodiale la plus répandue sur tous les continents, excepté en Afrique subsaharienne où *P. falciparum* prédomine, malgré la présence de *P. malariae* qui peut parfois y causer jusqu'à 1/3 des cas de paludisme et celle de *P. ovale*, néanmoins plus rare (Mendis K. et al., The Neglected Burden of Plasmodium vivax Malaria, Am. J. Trop. Med. Hyg., 2001, 64 (1-2 suppl) : 97-106).

Plus précisément, le parasite *P. vivax* se situe majoritairement en Asie du Sud-est et dans le Pacifique où il serait responsable de 49 % des cas du paludisme, mais également, dans une moindre mesure, dans les territoires d'Afrique de l'Est et du Sud. Il est en fait présent dans les populations afro-asiatiques, notamment au Kenya, en Tanzanie ainsi que dans les îles de l'Océan Indien, comme par exemple à Madagascar. Par ailleurs, la prévalence de cette espèce augmente en Amérique du Sud et Amérique Centrale, avec de 71 à 81 % des cas de paludisme. On le retrouve notamment au Pérou, en Bolivie et en Guyane Française. Ce sont également 81 % des cas de paludisme qui sont imputables à *P. vivax* dans les régions méditerranéennes de l'Est et 100 % dans les pays de l'ex-URSS.

Les estimations concernant la prévalence géographique de ce parasite varient selon la méthodologie utilisée, mais une chose est sûre, l'importance de ce parasite a été largement sous-estimée.

Concernant le développement du parasite, les quatre espèces précitées sont toutes transmises *via* une piqure de moustique anophèle femelle. Une fois dans l'hôte humain, le parasite gagne les cellules hépatiques, y subit une phase de réplication asexuée et conduit à la formation de vésicules, les schizontes. Les vésicules ainsi formées sont libérées dans les sinusoïdes hépatiques pour rejoindre ensuite la circulation sanguine et y répandre un flot de jeune mérozoïtes pré-érythrocytaires prêts à infecter les globules rouges. Les jeunes mérozoïtes pré-érythrocytaires pénètrent à l'intérieur des globules rouges et débutent le cycle érythrocytaire. Les divisions successives des mérozoïtes provoquent l'éclatement des globules rouges parasités. Ce sont ces éclatements brutaux et synchrones qui sont à l'origine des accès de fièvre. Les mérozoïtes libérés dans la circulation sanguine infectent de nouveaux globules rouges ou érythrocytes. C'est le début du cycle asexué érythrocytaire ou schizogonie érythrocytaire. Après l'invasion des érythrocytes par les mérozoïtes, le développement du parasite commence par le stade anneau, puis il évolue en forme trophozoïte.

Par ailleurs, il est à noter que pour les espèces autres que *P. falciparum*, certains mérozoïtes pré-érythrocytaires ne gagnent pas le sang directement mais s'attaquent à de nouveaux hépatocytes.

Ces formes hépatiques, appelés hypnozoïtes, vont rester à l'état latent pendant une durée propre au type de souche et dépendant de son environnement. Elles vont entretenir dans le foie la parasitose pendant 2 ou 3 ans pour *P. ovale*, 3 à 5 ans ou plus pour *P. vivax* et pendant la vie entière pour *P. malariae*, avant de se réactiver en vagues successives, provoquant une forte fièvre, encore appelée fièvre tierce bénigne, l'une des formes du paludisme encore appelée malaria.

Au regard de cette spécificité, il est manifeste que des actifs, efficaces pour traiter des infections causées par *P. falciparum* se révèlent d'une manière générale, en revanche, insuffisants en terme d'efficacité à l'égard des infections induites par ces autres espèces, dans la mesure où ils se bornent à éliminer les formes circulantes du parasite et n'agissent aucunement sur les formes quiescentes stockées dans les hépatocytes humains.

Or, les traitements antipaludiques conventionnels à disposition pour traiter l'ensemble des infections parasitaires du genre *Plasmodium* sont des traitements dont l'efficacité n'a été pour l'essentiel validée que sur l'espèce *P. falciparum.*

Ainsi, la chloroquine a été et reste le traitement de première intention du paludisme à *P. vivax* depuis 1946. Elle est souvent recommandée avec un relais par une 8-amino-quinoléine, la primaquine (World Health Organization, « Annex 10. Treatment of Plasmodium vivax, P. ovale and P. malariae infections" 01/01/06, Guidelines for the treatment of malaria, p. 225-239).

Toutefois, les phénomènes de résistance de *P. vivax* à la chloroquine rendent cet actif de moins en moins efficace contre ce parasite.

Pour ce qui est de la primaquine, elle présente d'importants problèmes de toxicité et provoque un risque accru d'hémolyse chez des sujets déficitaires en glucose-6-phosphate-déshydrogénase. Ces sujets atteints de déficit en glucose-6-phosphate-déshydrogénase sont souvent originaires de l'Afrique, le Moyen-Orient, l'Inde, le bassin méditerranéen ou le Sud-Est de l'Asie.

D'autres composés, comme la quinine, la chloroquine, la méfloquine et les dérivés d'artémisinine ont peu ou pas d'effets sur la forme hépatique du parasite.

Les antifolates et l'atovaquone, utilisés initialement en combinaison pour le traitement du parasite dans sa phase circulante, ont aussi été reconnues pour être actives sur les hépatocytes. Cependant, de nombreuses résistances à ces actifs sont apparues.

Quant aux ACT (Artemisinine based-Combination Therapy), très peu d'études ont, à ce jour, été consacrées à leur efficacité sur *P. vivax.*

Il n'existe donc pas de réels traitements efficaces à ce jour pour traiter et prévenir les infections par *P. vivax* et plus généralement pour traiter les formes hypnozoïtes latentes hépatiques, caractéristiques des rechutes.

En conséquence, il demeure à ce jour un besoin pour un traitement préventif et curatif spécifique des infections causées par un parasite du genre *Plasmodium,* dont le cycle de vie comprend une phase de latence hépatique chez l'hôte humain.

Ainsi, la présente invention décrit l'utilisation de la ferroquine ou de son métabolite N-déméthylé ou un de leurs sels pharmaceutiquement acceptables pour le traitement et/ou la prévention d'infections causées par un parasite du genre *Plasmodium,* dont le cycle de vie comprend une phase de latence hépatique chez l'hôte humain.

Les infections sont causées en particulier par *P. vivax*, *P. ovale* ou *P. malariae* et plus particulièrement par *P. vivax.*

La présente invention décrit donc la ferroquine ou son métabolite N-déméthylé ou un de leurs sels pharmaceutiquement acceptables pour son utilisation pour le traitement et/ou la prévention d'infections des cellules sanguines et/ou hépatiques infectées par un parasite du genre *Plasmodium,* en particulier *P. vivax*, *P. ovale* ou *P. malariae*, et plus particulièrement *P. vivax*.

La présente invention vise la ferroquine ou un de ses sels pharmaceutiquement acceptables pour son utilisation pour le traitement des infections des cellules sanguines causées par plasmodium vivax aux stades de développement parasitaire anneau et trophozoïte mature.

La présente invention décrit également la ferroquine ou son métabolite N-déméthylé ou un de leurs sels pharmaceutiquement acceptables pour son utilisation pour le traitement, la prévention et l'élimination des formes hypnozoïtes quiescentes stockées dans les hépatocytes humains, notamment issues d'infections causées par un parasite du genre *Plasmodium,* en particulier *P. vivax*, *P. ovale* ou *P. malariae*, et plus particulièrement *P. vivax.*

La présente invention décrit aussi la ferroquine ou son métabolite N-déméthylé ou un de leurs sels pharmaceutiquement acceptables, pour son utilisation pour la prévention des rechutes dues à une infection causée par le parasite P. *vivax.*

La présente invention mentionne aussi la ferroquine ou son métabolite N-déméthylé ou un de leurs sels pharmaceutiquement acceptables, pour son utilisation pour le traitement et/ou la prévention de fièvres tierces bénignes dues à une infections causées par *P. vivax..*

La présente invention a également pour objet la ferroquine ou un de ses sels pharmaceutiquement acceptables, pour son utilisation pour le traitement d'infections causées par *P. vivax* chez des patients déficitaires en glucose-6-phosphate-déshydrogénase.

Cette ferroquine ainsi que d'autres dérivés apparentés se différenciant de celle-ci au niveau des substituants figurant sur le cycle quinoléine, sont décrits dans WO 96/35698.

Structurellement, elle résulte de l'insertion d'un groupement ferrocène à l'intérieur d'une molécule de chloroquine et correspond à un composé de structure qui suit :

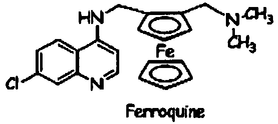

Son métabolite (N)-déméthylé correspond à un composé de structure qui suit :

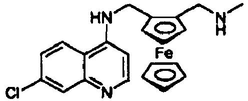

Les souches parasitaires spécifiquement visées dans WO 96/35698 sont les souches *P. falciparum.* En effet, l'élaboration de tels complexes repose précisément sur l'affinité de ce parasite pour le fer présent dans les hématies qu'il infecte.

La ferroquine est un agent antipaludéen connu ayant une activité supérieure à celle de la chloroquine dans divers parasites du genre *Plasmodium* (Barends et al, "In vitro activity of ferroquine (SSR 97193) against P. falciparum isolates from the Thai-Burmese border" Malaria journal, Biomed central, vol. 6, no. 1, p. 81 ; Fouda et al, "On the medicinal chemistry of ferrocene" Database biosisBiosciences information service ; Olliaro et al, "The global portfolio of new antimalarial medicines under development" Clinical pharmacology an therapeutics, vol. 85, no. 6, p. 584-595).

L'activité *in vitro* de la ferroquine sur des souches de *P. falciparum* résistantes à la chloroquine est indiquée ; des études complémentaires sont cependant nécessaires (Barends et al, "In vitro activity of ferroquine (SSR 97193) against P. falciparum isolates from the Thai-Burmese border" Malaria journal, Biomed central, vol. 6, no. 1, p. 81).

La présente demande démontre que la ferroquine s'avère particulièrement efficace pour le traitement d'infections par *P. vivax.* Contrairement à la chloroquine, et de façon tout à fait inattendue, elle permet non seulement d'éliminer le parasite dans sa phase circulante au niveau des cellules sanguines, mais elle le fait avec une égale efficacité sur les formes trophozoïtes immature (« stade anneau ») et mature de *P. vivax.*

Les actifs considérés selon la présente invention peuvent se présenter sous forme de base libre, mais également sous forme de sel, d'hydrate ou de solvat (ces derniers étant définis comme des associations ou des combinaisons de la ferroquine avec, respectivement, une ou plusieurs molécules d'eau ou de solvant). Au titre de sels convenant à la présente invention, on peut par exemple citer les sels tartrate, L-tartrate, di-tartrate, ou di-chlorhydrate.

Avantageusement, on utilise la ferroquine sous forme de base libre.

L'invention se rapporte à une composition pharmaceutique comprenant, en tant que principes actifs, la ferroquine ou son métabolite N-déméthylé ou un de leurs sels pharmaceutiquement acceptables, pour son utilisation pour le traitement d'infections par *P. vivax.*

Une telle composition pharmaceutique contient des doses thérapeutiquement efficaces de ferroquine ou de son métabolite (N)-déméthylé, ou l'un de leurs sels pharmaceutiquement acceptables, de leurs hydrates ou de leurs solvats de la ferroquine ainsi qu'au moins un excipient pharmaceutiquement acceptable. Lesdits excipients sont choisis selon la forme pharmaceutique et le mode d'administration souhaité, parmi les excipients habituels qui sont connus de l'Homme du métier.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

Des voies d'administration préférées sont les voies orale, rectale et injectable.

Par exemple, lorsqu'on prépare une composition solide sous forme de comprimés, on mélange les ingrédients actifs avec un ou plusieurs excipients pharmaceutiques, tels que la gélatine, l'amidon, le lactose, le stéarate de magnésium, le talc, la silice, la gomme arabique, le mannitol, la cellulose microcristalline, l'hydroxypropyl-méthylcellulose, le croscarmellose, le stéarate de magnésium, l'hypromellose, ou analogues. On peut enrober les comprimés de saccharose, d'un dérivé cellulosique ou d'autres matières adaptées à l'enrobage. Les comprimés peuvent être réalisés par différentes techniques, telles que la compression directe, la granulation sèche, la granulation humide ou la fusion à chaud.

On peut également obtenir une préparation sous forme de gélules en mélangeant les ingrédients actifs avec un diluant et en versant le mélange obtenu dans des gélules molles ou dures.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylène glycol ou le butylène glycol.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

A titre d'exemple, une forme unitaire d'administration de la ferroquine sous forme de comprimé peut comprendre les composants suivants :
Ferroquine 50 mg
Mannitol 224 mg
Croscaramellose sodique 6 mg
Amidon de maïs 15 mg
Hydroxypropyl-méthylcellulose 2 mg
Stéarate de magnésium 3 mg

### Evaluation in vitro de l'efficacité de la ferroquine versus d'autres antipaludiques de référence, sur des isolats sanguins infectés par P. vivax

Le test qui suit est réalisé sur 110 isolats de *P. vivax,* collectés dans le Nord-Ouest de la Thaïlande (Province de Tak) sur des patients consentants, infectés par *P. vivax* et en phase aigüe de l'infection, venant consulter à l'Unité de Recherche Malaria de Shoklo (SMRU).

Les prélèvements sont collectés 5 heures après leur arrivée et placés dans des tubes héparinisés de 5 ml, à température ambiante.

La parasitémie des isolats collectée est d'environ 4432 parasites/µl. 99 isolats sont finalement cultivés avec succès et parmi eux, seuls sont retenus ceux présentant plus de 80 % de trophozoïtes à l'observation microscopique.

Les plaquettes et les leucocytes sont retirées de ces isolats, par analogie avec la méthode décrite dans Kanlaya et al., Malaria Journal, 2009, 8:115.

Une soixantaine d'échantillons sont finalement retenus et une dizaine d'isolats par plaque ou par expérience est testée en duplicata.

L'activité des différents actifs est testée dans des puits prédosés, par analogie avec la méthode décrite dans Barends et al., Malaria Journal 2007,6:81 et Brice et al., Antimicrobial agents and chemotherapy, Jan 2003, pp 170-173.
La Figure 1 présentant un diagramme dose/réponse, illustre les résultats de CE50 (concentration effective 50) obtenus pour la ferroquine et des antipaludiques de référence, la chloroquine, la méfloquine et la pipéraquine.
La Figure 2 présentant un diagramme dose/réponse, illustre les résultats de CI50 (concentration inhibitrice 50) obtenus pour la ferroquine et la chloroquine.

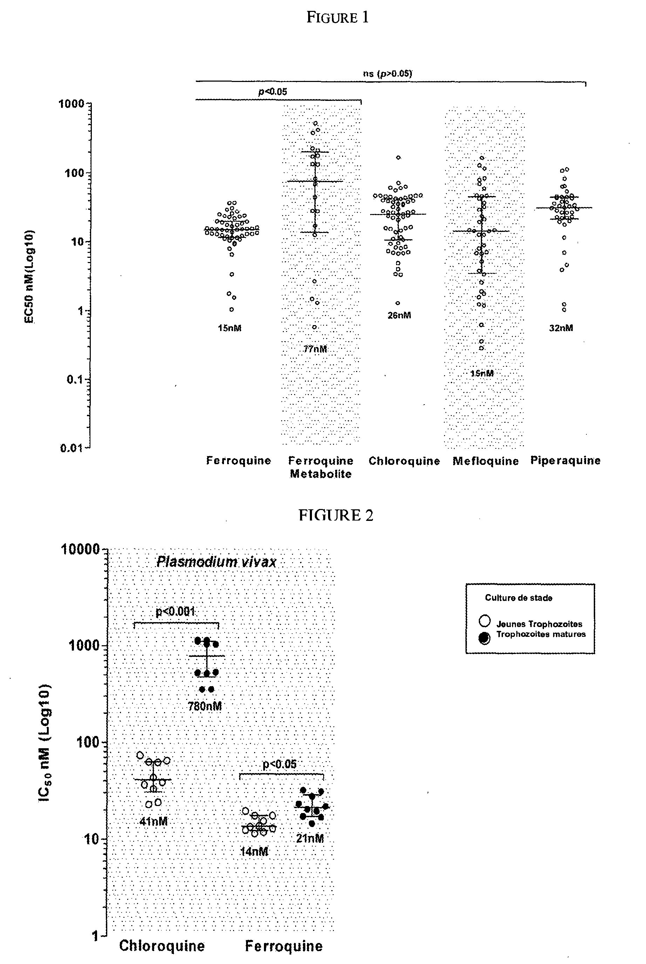

Comme indiqué sur la figure 1 précitée, la CE50 la plus faible, de l'ordre de 6 nM, est celle de la ferroquine, démontrant ainsi son efficacité accrue par rapport aux antipaludiques classiques.

Comme indiqué sur la figure 2 précitée, la ferroquine est testée pour son activité sur les stades trophozoïtes matures et les jeunes trophozoïtes immatures ("stade anneau") de *P. vivax.* La chloroquine est 10 fois à 20 fois moins actives sur les stades trophozoïtes matures que sur les stades anneaux comme également décrit dans Russell, B. et al. 2008, Antimicrob. Agents Chemother. 52:1040-5. En revanche et de façon tout à fait inattendue, la ferroquine est active sur les deux stades du parasite testés avec des activités comparables de 14 et 21nM pour les stades anneau et trophozoïte mature respectivement. Ceci démontre un potentiel d'activité de la ferroquine sur différents stades de développement de *P. vivax,* supérieur à celui de la chloroquine et donc une avancée thérapeutique par rapport à la chloroquine.

## Revendications

1. Ferroquine ou un de ses sels pharmaceutiquement acceptables pour utilisation dans le traitement d'infections des cellules sanguines causées par *Plasmodium vivax* aux stades de développement parasitaire du trophozoïte immature « stade anneau » et du trophozoïte mature.

2. Ferroquine ou un de ses sels pharmaceutiquement acceptables pour utilisation selon la revendication 1, **caractérisée en ce que** le traitement est le traitement d'infections chez des patients déficitaires en glucose-6-phosphate-déshydrogénase.

## Patentansprüche

1. Ferroquin oder eines seiner pharmazeutisch annehmbaren Salze für die Verwendung zur Behandlung von Infektionen von Blutzellen, die durch *Plasmodium vivax* in den Parasitenentwicklungsstadien des unreifen Trophozoiten, des so genannten "Ringstadiums", und des reifen Trophozoiten hervorgerufen werden.

2. Ferroquin oder eines seiner pharmazeutisch annehmbaren Salze für die Verwendung nach Anspruch 1, **dadurch gekennzeichnet, dass** es sich bei der Behandlung um die Behandlung von Infektionen bei Patienten mit einem Glucose-6-phosphatdehydrogenase-Mangel handelt.

## Claims

1. Ferroquine or a pharmaceutically acceptable salt thereof, for use in the treatment of infections of blood cells caused by *Plasmodium vivax* at the parasitic growth stages of the immature trophozoite "ring stage" and of the mature trophozoite.

2. Ferroquine or a pharmaceutically acceptable salt thereof for use according to Claim 1, **characterized in that** the treatment is the treatment of infections in patients deficient in glucose-6-phosphate dehydrogenase.
